# EUROPEAN PATENT APPLICATION

(11) **EP 0 989 134 A1**
(43) Date of publication of application: **29.03.2000**
(21) Application number: 98203051.2
(22) Date of filing: 11.09.1998
(51) Int. Cl.: C07F 5/00, C07F 5/06, C08F 10/00, C07C 2/54, B01J 31/30

(54) **Process for the in situ preparation of an ionic liquid**

(71) Applicant: Akzo Nobel N.V., 6824 BM Arnhem (NL)
(72) Inventor: Lacroix, Christine Pascale Mireille, 7391 WK Twello (NL); Dekker, Francisca Hildegonda Maria, 6843 VN Arnhem (NL); Talma, Auke Gerardus, 7437 AT Bathmen (NL); Seetz, Johannes Wilhelmus Franciscus, 7558 JB Hengelo (NL)
(74) Representative: Schalkwijk, Pieter Cornelis

(57) **Abstract**

The invention relates to a process to prepare ionic liquids comprising a complex of metal halide and an ammonium halide. According to this process, a dry amine and a dry hydrohalic acid are reacted with a metal halide to form the desired ionic liquid. The process can be conducted at atmospheric pressure and at temperatures below 100°C, preferably in the presence of a heel of the same or another ionic liquid. The resulting ionic liquid can be used without further purification in conventional applications.

## Description

This invention relates to a process to prepare ionic liquids comprising a complex of a metal halide and an ammonium halide.

Such ionic liquids are known from, for instance, EP-A-0 576 323. In this patent application it is disclosed how first a pyridine, picolin-4 or N-methyl imidazole salt of hydrochloric or hydrobromic acid is prepared, after which the pure amine hydrohalide salt is contacted with a metal halide to form the ionic liquid.

Similarly, WO 95/21871 discloses how metal-containing ionic liquids are produced by first reacting a 1-alkylimidazole with a 1-haloalkane to make a 1,3-dialkyl substituted imidazolium halide, which is then contacted with a metal halide to form an ionic liquid.

In these processes the ammonium halide is first prepared, then separated and/or purified, and subsequently contacted in the pure form with the metal halide. The ammonium halides typically are hygroscopic salts often obtained as viscous oils. Such hygroscopic salts are difficult to handle. Also, they often include various side products that need to be removed. Consequently, these oils typically are treated to obtain the ammonium halide in the pure form. This handling and purification, also because of said hygroscopy, is cumbersome. Also, very long reaction times have been reported for some syntheses, which is undesired.
Furthermore, contacting the resulting ammonium halide salt with the metal halide to form the corresponding ionic liquid typically takes place in a solvent to avoid an inhomogeneous mixture being formed. Such a solvent may be undesired, depending on the intended use of the ionic liquid. Therefore, further processing steps generally are required in order to obtain the desired ionic liquid in an acceptable form.

Because of growing interest in the use of ionic liquids, there is a need for an improved process to prepare them. In comparison with the known processes, the desired process should be less elaborate, result in less waste, and lead to products with high purity in good yields.

The process according to the invention is such an improved process. The process is characterized in that a dry amine is contacted with a dry halide donor in the presence of a dry metal halide.

Preferably, said reaction is conducted in a heel of an ionic liquid. The term "heel" refers to the fact that a predetermined and preformed amount of ionic liquid is used in the process. Typically, such a heel is the residue of a previous reaction batch. However, for the purpose of this application, the term heel is used for any ionic liquid that is added to the process wherein amine, halide donor, and metal halide are reacted. Also, said heel may be present in the reactor before the reactants are added thereto or not until after said reactants have been added, as well as during the course of the reaction. The ionic liquid in said heel can serve as a (co)solvent and catalyst for the reaction of amine, halide donor, and metal halide reactants. The ionic liquid of said heel may be the same as the prepared ionic liquid, but may also be a different ionic liquid. Preferably, the ionic liquid of the heel is the ionic liquid formed in the process. If the heel of ionic liquid is not present when the reactants are all introduced into the reactor, then it is preferred to use another (co)solvent, which is to be introduced into the reactor ahead of said reactants in order to allow a more efficient heat transfer to the reactor and a more homogeneous reaction mixture. However, said (co)solvent may also be used in combination with said heel of ionic liquid in order to, for instance, adjust the viscosity of the reaction mixture, improve the reaction ratio, improve the heat transfer throughout the reaction mixture, etc. If a (co)solvent is used, then it preferably is a solvent which is acceptable or desirable in the process where the ionic liquid is used. For example, if the ionic liquid is intended for use in alkylation processes of benzene, then the preferred solvent is benzene.

In the claimed process, some dry metal halide must be present in the reactor when the dry amine and the dry halide donor are contacted. Accordingly, one can add all three components to the reactor at the same time in a certain rate, e.g., by using a screw feeder for the metal halide and introducing the halide donor and the amine in the gas or liquid form. This is a preferred mode of operation. Also, each of the components can be introduced independently, either continuously or discontinuously or both, e.g., by introducing a portion of a reactant before the contacting step and dosing the remainder in a continuous fashion. If the metal halide is to be formed "in situ," see below, then it is preferred to first introduce the metal and the hydrohalic acid, followed by the addition of the other halide donor and the amine. In a preferred embodiment, the amine and the halide donor are dosed to the reactor containing part or all of the metal halide that is desired for making the ionic liquid. By varying the dosing rates of the amine and/or the halide donor, the reaction can be controlled. If not all metal halide is introduced into the reactor before the addition of the amine and the halide donor commences, then the remainder of the metal halide can be introduced while the other reactants are dosed or thereafter. If dosed thereafter, then this may be done in a subsequent reactor. However, reaction in one and the same reactor is preferred. Also, preferably more than 10 percent by weight (%w/w), more preferably more than 25 %w/w, and most preferably more than 50 %w/w of all metal halide is present in the reactor before the amine and the halide donor are dosed.

The term "ionic liquid" is used to denominate all materials that are liquid at a temperature below 100°C at atmospheric pressure and are fused salt compositions comprising a mixture of a metal halide and an alkyl-containing amine salt. Preferably, the ionic liquid is liquid at temperatures below 60°C, more preferably below 30°C, at atmospheric pressure.

The reaction according to the invention, when optimized, is almost quantitative. Typically, the reaction temperature is between ―20°C and 170°C. Preferably, the temperature is selected between 25°C and 150°C. The reaction temperature can be controlled within the desired range by using common heating and cooling means or by adjusting the dosing rates of the reactants. The pressure during the reaction is not very critical and can be selected in the range of 0.5 to 50 bar. Preferably, a pressure is selected in the range of 1 to 20 bar. More preferably, the pressure is kept between atmospheric (1 bar) and 5 bar. Depending on the nature of the reactants, the reaction may be exothermic or endothermic.

Preferably, the reaction is carried out in an inert (dry) atmosphere because of the hygroscopic nature of reactants and products. Typically, such an inert atmosphere is obtained by flushing the reactor with an appropriate gas, such as dried air, nitrogen, argon and/or helium. Most preferred is the use of dried air or nitrogen.

The amine reactant may be any amine compound or mixture of amine compounds suitable for the preparation of ammonium salts. Preferred amine compounds useful in this invention have at least one alkyl substituent, but may contain as many as three alkyl substituents on the nitrogen atom. The alkyl substituents of the amine may form ring systems, in particular a monocyclic or bicyclic ring. Also, one or two of the substituents may be of an aromatic nature, such as aryl groups, but this is not preferred. The alkyl and aryl groups may be further substituted with additional groups, for instance, with alkyl and/or aryl groups. Also, the amine compound substituents may contain one or more hetero atoms, in particular nitrogen and/or oxygen. In order to obtain an ionic liquid with acceptable viscosity, however, the substituents preferably are limited to types with fewer than 30, preferably fewer than 20 carbon atoms. Such compounds may be represented by the formula: 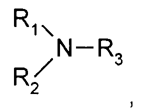 wherein
R₁₋₃ are independently selected from H, with the exception that not all three of R₁₋₃ are H, and linear or branched C₁₋₃₀ hydrocarbyl, optionally containing further O and/or N atoms, while R₁ and R₂ may be linked to form a cyclic structure.

Typical examples of suitable amine compounds include: all trialkyl amines, such as trimethyl amine, triethyl amine, tri-n-propyl amine, triisopropyl amine, tributyl amine, and tricyclohexyl amine, mono- and di-methyl amines, dibutyl amine, ethylenediamine, diethylenetriamine, triethylenediamine, N,N-dimethylethyl amine, N,N-dimethylpropyl amine, N,N-diethylmethyl amine, N,N-diphenylmethyl amine, N-phenyl-N-methylethyl amine, N,N,N',N'-tetramethyldiamino ethane, guanidine, substituted guanidines, pyridine, substituted pyridines, picolines, lutidines, piperazine, quinoline, piperidines, N-alkyl imidazoles, and N-alkyl imidazolines. Preferred are trialkyl amines, pyridine, and N-alkyl imidazoles. More preferably, the amine is selected from trimethylamine, triethylamine, dimethylamine, monomethylamine, N-methyl imidazole, pyridine, 1,4-diazabicyclo-[2,2,2]-octane(Dabco®), N-methyl-1,4-diazabicyclo-[2,2,2]-octane, and mixtures thereof. Most preferred Is the use of amines selected from trimethylamine, triethylamine, dimethylamine, monomethylamine, N-methyl imidazole, pyridine, and mixtures thereof.

The halide donor is selected from hydrohalic acids, halo substituted methanes, and mixtures thereof. Halo substituted alkyls with more than one carbon atom were found to react with the amines at an unfavourable rate. The halo atoms in these compounds are selected from chloro, bromo, iodo, and fluoro. Preferred is the use of monochloro or monobromo compounds such as HCl, HBr, CH₃Cl and CH₃Br. More preferred halide donors are HCl and CH₃Cl, the former being most preferred.

The metal halide that is useful in this invention can form anions containing polyatomic halide bridges in the presence of an ammonium salt. Suitable metal halides include those selected from halides of Group 8, Group 1b, Group 2b, and Group 3a metals of the Periodic Table of Elements. Preferably, the metal is selected from aluminium, gallium, indium, iron, copper, zinc, and mixtures thereof. Particularly preferred metal halides are selected from iron trihalide, copper monochloride, zinc dichloride, aluminium trihalide, alkylaluminium dihalide, dialkylaluminium halide, and mixtures thereof. Preferably the halide in these metal halide compounds is chlorine, bromine, or a mixture thereof, as, for instance, in ferri trichloride and aluminium bromide. Most preferred are aluminium halides where the halide is predominantly chlorine, i.e. aluminium trichloride. For the purpose of this invention, it is to be understood that the metal halide can be introduced into the reaction, or else formed "in situ" by contacting a hydrohalic acid, which preferably is also used as halide donor in the reaction with the amine, with the desired metal. The resulting metal halide can subsequently react with additional halide donor and amine to form the desired ionic liquid.

The molar ratio of amine to metal halide in the ionic liquid generally ranges from about 2:1 to 1:3. More preferably, the molar ratio is between 1:2 and 2:1. Most preferred is an ionic liquid derived from an amine selected from trimethylamine, N-methyl imidazole, N-ethyl imidazole, and pyridine; hydrochloric acid; and aluminium trichloride, with the molar ratio of amine to aluminium trichloride being between 1:2 and 2:1.

The molar ratio of amine to halide donor is from 2:1 to 1:2. More preferably, the molar ratio is between 3:2 and 2:3. Most preferably, these reactants are used in about equimolar amounts. However, if a metal is used to prepare the metal halide in situ, then the amount of halide donor needed for this reaction is to be adapted additionally.

Although the preferred process according to the invention uses only the ionic liquid as a solvent, a (co)solvent may be used if so desired, e.g., to reduce the hygroscopic nature of the product or to improve the heat transfer. If used, the (co)solvent preferably leads to phase separation in the product, so that it can be removed without undue effort, or it is selected from solvents that are acceptable, or even desired, in the process where the ionic liquid is used. The (co)solvents should not react with any of the reactants. Examples of (co)solvents that can be used are benzene, toluene, xylene, pentane, hexane, cyclohexane, nonane, decane, dodecane, and other, preferably aliphatic, hydrocarbons. Typically, said (co)solvent is selected on the basis of its boiling point to ensure that no safety hazard occurs near or at the reaction temperature and pressure. Therefore, the higher-boiling (co)solvents (boiling point above 150°C at atmospheric pressure) are preferred for reactions above 100°C at or near atmospheric pressure. However, the skilled man will have no problem finding other suitable solvents.

The reaction can be carried out in a batch-type process but is also very suitable for continuous operation, after modifications well known to the artisan.

The ionic liquid can be used without further purification in the known applications. However, if so desired, the known purification methods such as filtration and distillation may be used to remove impurities and unreacted raw materials.

Especially when further purification is refrained from, the total reaction and handling time is short when compared to processes according to the prior art, while hardly any waste is produced. This gives very high reactor (space-time) yields and is, therefore, very economical. Typically, the process has a yield of ionic liquid greater than 90% after 4 hours reaction time. However, depending on the reactants used, yields of ionic liquid of greater than 95% after 4 hours reaction time have been observed. Most preferred is the process according to the invention where more than 99% yield of ionic liquid is obtained in less than 4 hours, preferably less than 2 hours.

If in the process a heel of an ionic liquid is present when the reactants are added or dosed, this heel can serve as a solvent but also as a catalyst. The solvent effect ensures that the reaction mixture remains easily stirrable liquid throughout the process. If they are dosed, acceptable dosing rates of the reactants will depend, inter-alia, on the reactor size, the reaction temperature, and the size of the heel that is present in the reactor. The skilled man will have no problem optimizing the dosing rate on the basis of the information disclosed herein.

Typically, the ionic liquids according to the invention may be used as electrolytes in batteries, photo-electrochemical cells, electro-refining, and electroplating processes. However, they are also suitable as catalysts in chemical reactions such as Friedel-Crafts alkylation and acylation processes, and oligomerization or polymerization processes. Furthermore, they can act as solvents in specific chemical processes, especially as solvents for organometallic complexes. The ionic liquids as obtained by the process according to the invention are particular useful as catalysts in the aforementioned chemical processes, i.e. in the production of linear alkyl benzenes.

The invention is further illustrated by the following examples.

### Comparative Example A

Of a commercially available 70% aqueous solution of trimethylamine hydrochloride (ex Akzo Nobel), 200 g were placed in a porcelain dish and air-dried until 40% by volume of the liquid had evaporated. A slurry resulted. The remaining moisture in the slurry was removed by vacuum distillation. In a nitrogen atmosphere, the formed solid trimethylamine hydrochloride was mixed, with stirring, with two molar equivalents of aluminium trichloride powder in two steps. The reaction was exothermic. After heating to 130°C for one hour, a brown ionic liquid was formed.

### Comparative Example B

In a glove box with a nitrogen atmosphere, a flask, equipped with a stirrer, was charged with 2.3 g (16.7 mmoles) of dry triethylamine hydrochloride, obtained by reacting triethylamine and dry hydrochloric acid in dry ether, filtration of the white precipitate, washing with dry ether (all using a nitrogen atmosphere), and drying of the product under vacuum. The ammonium salt was reacted with 4.4 g (33.4 mmoles) of aluminium trichloride by adding the aluminium trichloride powder portion-wise, using a spatula. The reaction mixture immediately became sticky and did not become completely fluid until 50% of the aluminium trichloride had been added. The reaction temperature was controlled at 30°C using an ice bath. The product was an ionic liquid of slightly brownish colour.

### Comparative Example C

Inside a glove box with a nitrogen atmosphere a flask, equipped with a stirrer, was charged with 2.5 g (15.1 mmoles) of dibutylamine hydrochloride, which was obtained as described in Comparative Example B with dibutylamine substituting for the triethylamine. The ammonium salt was reacted with 4.0 g (30.0 mmoles) of aluminium trichloride powder by adding the aluminium trichloride portion-wise using a spatula. The reaction mixture immediately became sticky and did not become completely fluid until 50% of the aluminium trichloride had been added. The reaction temperature was controlled at 30°C using an ice bath. The product was an ionic liquid of slightly brownish colour.

### Example 1

Into a stirred, cooled reactor of 1 liter with a nitrogen atmosphere, 345 g of an ionic liquid were introduced. The ionic liquid was obtained by reacting 117 g of purified and dried trimethylamine chloride and 328 g of AlCl₃ according to Comparative Example A. To this ionic liquid, 52.8 g of AlCl₃ were added. Thereafter, while stirring, 57.4 g of dry trimethylamine gas and 35.5 g of dry hydrochloric acid gas were added simultaneously over 3 hours at atmospheric pressure. The temperature of the reaction mixture was controlled between 30 and 80°C. After adding another 205 g of AlCl₃ and heating to 135°C for one hour, a brownish trimethylammonium chloroaluminate ionic liquid according to the invention was obtained.

### Examples 2 and 3

In a glove box with a nitrogen atmosphere, 1.38 mmoles of the ionic liquid of Example 1 were mixed with 12.5 g of benzene at room temperature (20-25°C) in a 50 ml three-necked flask equipped with a stirrer. Then 8.31 mmoles of an alkylating agent were added using a syringe. After 10 minutes the reaction was found to be complete. The reaction product was analyzed for the amount of mono-, di-, and tridodecyl benzene, and the weight distribution of the resulting monododecyl benzene isomers was determined by conventional means. The results are compiled in the following table.

| Example | alkylating agent | product (area%) | | | Isomer distribution of monododecyl benzene (area%) | | | | |
|---|---|---|---|---|---|---|---|---|---|
| | | Mono | di | tri | A | B | C | D | E |
| 2 | Dodecyl chloride | 95.3 | 3.2 | 0 | 23.7 | 11.7 | 14.0 | 24.2 | 21.5 |
| 3 | 1-dodecene | 83.0 | 9.8 | 4.0 | 20.2 | 10.8 | 16.2 | 35.8 | 0 |
| A=6- and 5-dodecyl benzene B=4-dodecyl benzene C=3-dodecyl benzene D=2-dodecyl benzene E=1-dodecyl benzene | | | | | | | | | |

These examples show the suitability of the ionic liquid obtained in a process according to the invention for use as a catalyst in an alkylation reaction.

### Example 4

To a 100 ml three necked flask with stirrer and nitrogen atmosphere and containing 5.15 g of dry 1-methylimidazole, 16.7 g dry aluminium chloride was added portion-wise during 30 minutes. While adding the aluminium chloride, a gas mixture of 50 % by volume of dry HCl and 50 % by volume of nitrogen was bubbled through the reaction mixture. After 15 minutes the temperature had risen to 80°C and the reaction mixture became solid. In order to proceed with a liquid reaction mixture, the mixture was heated to 100°C. After having dosed all aluminium chloride, also the addition of HCl was stopped and the reaction mixture was cooled. The resulting ionic liquid was liquid at 25°C. Analysis by ¹H-NMR (60MHz, no solvent) confirmed the expected structure (5.4 ppm (3H,s), 8.8 ppm (2H,m), 9.9 ppm (1H,s)).

## Claims

1. Process to prepare an ionic liquid comprising a complex of a metal halide and an ammonium halide, characterized in that a dry amine is reacted with a dry halide donor in the presence of dry metal halide.

2. Process according to claim 1 wherein an ionic liquid is present during the whole of said reaction.

3. A process according to claim 2 wherein the ionic liquid is of the same material as the ionic liquid being produced.

4. A process according to any one of the preceding claims wherein the reactants are contacted at a temperature of ―20°C to 170°C at a pressure of 0.5 to 5 bar.

5. A process according to any one of the preceding claims wherein the gas phase above the liquid reaction mixture comprises an inert gas.

6. A process according to any one of the preceding claims wherein the amine is selected from compounds with the structure 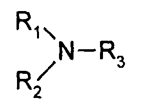 wherein R₁₋₃ are independently selected from H, with the exception that not all three of R₁₋₃ are H, and linear or branched C₁₋₃₀ hydrocarbyl, optionally containing further O and/or N atoms, while R₁ and R₂ may be linked to form a cyclic structure.

7. A process according to claim 6 wherein the amine is selected from trialkyl amines, ethylenediamine, diethylenetriamine, guanidine, substituted guanidines, 1―alkyl imidazoles, pyridine, substituted pyridines, picolines, lutidines, piperazine, quinoline, piperidines, and N-alkyl imidazolines.

8. A process according to claim 7 wherein the amine is selected from trialkyl amines, pyridine, and 1-alkyl imidazoles.

9. A process according to any one of claims 1-8 wherein the metal halide is a halide selected from the metals aluminium, gallium, indium, iron, copper, and zinc.

10. A process according to claim 9 wherein the metal halide is selected from iron trihalide, copper monohalide, zinc dihalide, aluminium trihalide, alkyl aluminium dihalide, and dialkylaluminium halide, aluminium trihalides being preferred.

11. A process according to claim 9 or 10 wherein the metal halide is a metal chloride.

12. A process according to any one of claims 9-11 wherein the metal halide is formed during and/or prior to the reaction of the amine and the halide donor, by reaction of the metal and dry hydrohalic acid.

13. A process according to any one of claims 1-12 wherein the molar ratio of amine to metal halide is between 2:1 and 1:3.

14. A process according to any one of claims 1-13 wherein the halide donor is hydrochloric acid or methylchloride.

15. A process according to any one of claims 1-14 wherein the molar ratio of amine to halide donor is between 2:1 and 1:2.

16. A process according to any one of claims 1-15 wherein a cosolvent is used.

17. Use of an ionic liquid resulting from a process according to any one of claims 1-16 as an electrolyte in batteries, photo-electro chemical cells, electro-refining, and electroplating processes, as a catalysts in chemical reactions such as Friedel-Crafts processes, and oligomerization or polymerization processes, and/or as a solvent in specific chemical processes.
